# EUROPEAN PATENT SPECIFICATION

(11) **EP 4 342 455 B1**
(45) Date of publication and mention of the grant of the patent: **14.10.2026**
(21) Application number: 23382958.9
(22) Date of filing: 22.09.2023
(51) Int. Cl.: A61K 9/00, A61K 9/08, A61K 31/00

(54) **PHARMACEUTICAL COMPOSITIONS AND MANUFACTURING METHODS THEREOF**
PHARMAZEUTISCHE ZUSAMMENSETZUNGEN UND HERSTELLUNGSVERFAHREN DAFÜR
COMPOSITIONS PHARMACEUTIQUES ET LEURS PROCÉDÉS DE FABRICATION

(30) Priority: 22.09.2022 EP 22382874; 28.04.2023 EP 23382405
(43) Date of publication of application: 27.03.2024
(73) Proprietor: Galenicum Health S.L.U., 08950 Esplugues de Llobregat (ES)
(72) Inventor: Pérez Pérez, María del Rocío, San Agustín de Guadalix (ES); Gómez Coello, Luis, San Agustín de Guadalix (ES)
(74) Representative: Galenicum Health S.L.U.

(56) References cited:
- WO-A1-2018/041739
- US-A1- 2022 265 674
- FDA: "PROVAYBLUE PRESCRIBING INFORMATION", 1 January 2016 (2016-01-01), pages 1 - 9, XP093294702, Retrieved from the Internet <URL:https://www.accessdata.fda.gov/drugsatfda_docs/label/2017/204630s005lbl.pdf>

## Description

### FIELD

The present invention relates to a pharmaceutical composition comprising methylene blue. Manufacturing processes of such compositions are also described.

### STATE OF THE ART

Methylthioninium chloride, also known as methylene blue or, 3,7-bis- (dimethylamino)-phenothiazin-5-ium chloride, is an oxidation-reduction agent used for the treatment of patients with acquired methemoglobinemia.

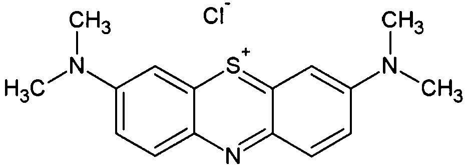

Methylene blue is an old known compound. According to Merck Index, 8th Edition (1964), it was first described in a German Patent in 1877 (Badische Anilin und Soda Fabrik, 1877).

Methylene Blue compounds used in the present invention can be obtained according to the processes disclosed in WO 2018/167185.

WO 2006/032879 and WO 2008/007074 disclose a high purity diaminophenothiazonium compound with a purity greater than 98%, an Azure B impurity content of less than 2% and a low content of certain metals. Moreover, this document discloses that in the different steps of the process, care is taken to use non-metallic materials, reagents and solvents free of metal residues and that the purification and hydration steps are performed in an enamelled reactor under nitrogen.

WO 2008/006979 discloses a method for preparing methylene blue with a low level of impurities and low metal content.

The European Public Assessment report for Methylthioninium chloride Proveblue discloses that methylene blue has a very strong affinity for metals and that it is difficult to manufacture a substance that complies with the requirements of Ph. Eur. Moreover, it discloses that methylene blue even absorbs metals from the brown vials where it is stored.

US 2022/0265674 discloses pharmaceutical composition of methylene blue comprising a pH adjuster. The incorporation of a pH adjuster in a pharmaceutical composition has certain drawbacks. Firstly, it can lead to undesirable chemical reactions that can negatively impact the efficacy and stability of the active ingredient. Additionally, the presence of a pH adjuster can increase the likelihood of microbial growth, which can compromise the safety of the product, something of great concern in a parenterally administered product. Moreover, adding manufacturing steps (and/or excipients) increases the cost of manufacturing a pharmaceutical product. Therefore, careful consideration should be given to the use of pH adjusters in pharmaceutical compositions to minimise potential disadvantages and ensure optimal performance and safety of the medication. The inventors have found out that avoiding traditional pH adjusters in a methylene blue composition has substantial benefits.

There is a need in the field of pharmaceutical manufacturing for a stable, safe and effective pharmaceutical composition of methylene blue wherein said methylene blue contains a higher level of impurities than the prior art.

### SUMMARY OF THE INVENTION

The present invention discloses pharmaceutical compositions comprising methylene blue and methods for preparing said compositions, wherein the active substance has significantly higher impurity levels and metal content than those identified in the prior art. The inventors have surprisingly found that a very specific pH is required in said pharmaceutical compositions in order to obtain a stable pharmaceutical composition.

The invention is directed to a sterile aqueous pharmaceutical composition consisting of methylene blue as active ingredient and water, wherein:
the Azure B impurity content of said composition ranges from 2.0% to 3.0%, the pH of said composition is from 3.2 to 3.9, preferably from 3.3 to 3.8 and the Iron (Fe) content of said pharmaceutical composition is above 20 ppm, preferably above 50 ppm, more preferably above 100 ppm, even more preferably above 120 ppm.

A second aspect of the invention deals with a process for the preparation of said pharmaceutical composition.

### DESCRIPTION

### Definitions

As used in the present disclosure, the following words, phrases, and symbols are generally intended to have the meanings as set forth below, except to the extent that the context in which they are used indicates otherwise.

All percentages are expressed by weight (w/w) unless specifically noted otherwise.

The term "active ingredient" as used herein refers to a therapeutically active compound, as well as any pharmaceutically acceptable hydrates and solvates of the compound.

In an embodiment, the term "consisting of" as used herein refers as to that no further features are present in the apparatus/method/product apart from the ones following said wording. It is considered that the methylene blue impurities such as Azure B are inherent to any methylene blue compound, as well as any other impurity (including metal impurities) that the API carries. Therefore, in the present application, the term "consisting of methylene blue as active ingredient and water" encompasses all methylene blue impurities.

The term "methylene blue impurities" include but are not limited to Azure A, Azure B, Azure C, metals and any unspecified degradation product from methylene blue or its impurities.

The term "pH adjuster" as used herein refers to pharmaceutically acceptable excipients which are added to the solution of the active agent to adjust the pH to a certain value. Such pH adjusters can be alkaline or acid agents and may comprise inorganic salts as well as organic acids or salts of organic acids. Additionally, the pH adjusters may be present in the form of a buffer.

The term "pharmaceutically acceptable" as used herein indicates that the substance or composition must be compatible chemically and/or toxicologically, with the other ingredients comprising a formulation, and/or the mammal being treated therewith.

The term "effective" amount as used herein refers to an amount of a compound, agent or substance that is of sufficient quantity to result in a quantifiable effect, e.g., a shift in the pH.

The term "solution" as used herein refers to a liquid preparation of one or more soluble chemical substances, which are dissolved in water.

The term "stable" as used herein refers to any parenteral solution of methylene blue as active ingredient having a sufficient physical and chemical stability to allow storage under any of the general storage conditions as defined by ICH Q1A (R2).

The term "suitable for parenteral administration" as used herein refers to a sterile solution that can be administered parenterally to a patient. Sterility is defined as the absence of living organisms. The conditions of the sterility test are given in the European Pharmacopoeia 10th edition.

The term "suitable pharmaceutical container" as used herein refers to a container for pharmaceutical use is an article which holds or is intended to contain and protect a drug and is or may be in direct contact with it. The container and its closure must not interact physically or chemically with the substance within in any way that would alter its quality.

The term "vial" as used herein refers to a vial suitable for containing injectable pharmaceutical compositions according to the European Pharmacopoeia 9th edition, Chapter 3.2.1 "Glass Containers for Pharmaceutical Use".

The term "terminal sterilization" as used herein refers to a process whereby a product is sterilized in its final container or packaging, which permits the measurement and evaluation of quantifiable microbial lethality. The probability of viable microorganisms being present on a product unit after exposure to the proper sterilization process should be less than 10⁻⁶.

The term "bioburden" as used herein refers to the population of viable microorganisms on or in raw materials, products, or labeling/packaging materials determined before sterilization. Bioburden analysis is carried out according to US Pharmacopoeia <71> Sterility tests.

The term "blanketing" as used herein refers to the act of maintaining an inert atmosphere of nitrogen gas (N₂) during storage and processing.

The term "0.2 µm pore filter", as used herein refer to filter where the nominal pore rating have been determined to be 0.2 µm or less, according to ISO 13408-2:2018 Aseptic processing of health care products.

Unless otherwise indicated, all the analysis methods are carried out according to the European Pharmacopoeia 10th edition and USP monograph for Methylene Blue Injection (USPNF 2022 Issue 1).

The terms "Azure B impurity" or "Azure B" as used herein refer to 3-(dimethylamino)-7-(methylamino)phenothiazin-5-ylium. Percentage of Azure B is determined by HPLC (Column XBridge Phenyl 100, flow rate 1.0 mL/min, UV detector wavelength of 246 nm, column temperature 30°C, mobile phase: 0.1% trifluoroacetic acid in water:acetonitrile (75:25% v/v), run time 15 minutes).

The term "Azure B impurity content" refers to the percentage amount of Azure B in the pharmaceutical composition with respect to the active ingredient (w/w).

The term "total impurity content" refers to the percentage amount of the sum of all the impurities in the pharmaceutical composition with respect to the active ingredient (w/w).

The terms "purity" or "chemical purity" referred to herein refers to the measure of an element containing a single substance without any other element tarnishing its standalone existence. In the present invention, the purity calculations have been provided based on the total organic impurity content and the metal content.

The terms "metal purity" or "metal content" referred to herein pertains to the amounts of the eleven (11) metals specified by the European Pharmacopoeia: Al, Cr, Zn, Cu, Fe, Mn, Ni, Mo, Cd, Sn, and Pb. The metal content is expressed relative to the amount of the active ingredient. The metal content has been determined following Ph. Eur. 10th Edition, chapter 2.4.20 - Determination of elemental impurities.

The inventors have found out that a sterile aqueous pharmaceutical composition comprising methylene blue as active ingredient wherein the active ingredient used to manufacture has not a high level of purity (total impurity content) as the methylene blue obtained as per the prior art can be surprisingly stable when the pH of said composition is below 4.0.

In a first aspect, the sterile aqueous pharmaceutical composition of the invention consists of methylene blue as active ingredient and water, wherein the Azure B impurity content of said composition ranges from 2.0% to 3.0%, the pH of said composition is from 3.2 to 3.9, preferably from 3.3 to 3.8 and the Iron (Fe) content of said pharmaceutical composition is above 20 ppm, preferably above 50 ppm, more preferably above 100 ppm, even more preferably above 120 ppm.

In an embodiment of the first aspect of the invention, said aqueous pharmaceutical composition comprises an Azure B impurity content ranging from 2.1% to 3.0%, preferably from 2.3% to 3.0%.

In another embodiment, the Azure B impurity content can be taken as representative of the total impurity content of the pharmaceutical composition.

In another embodiment, the pH of said pharmaceutical composition ranges from 3.3 to 3.8.

In another embodiment, the pharmaceutical composition of the invention is free from pH adjusters.

In another embodiment, the pharmaceutical composition of the invention is free from organic solvents. In a preferred embodiment, the solvent consists of water, more preferably, sterile water for injection.

In another embodiment, the said pharmaceutical composition has a concentration of 5 mg/mL of methylene blue or any of its hydrates.

In another disclosure, the metal content relative to the amount of methylene blue is above 20 ppm, preferably above 50 ppm, more preferably above 70 ppm, even more preferably above 100 ppm. In a further preferred embodiment, the content is above 120 ppm or above 130 ppm or above 150 ppm.

In another embodiment, the iron (Fe) content relative to the amount of methylene blue is above 20 ppm, preferably above 50 ppm, more preferably above 100 ppm, even more preferably above 120 ppm.

In another embodiment, said pharmaceutical composition is packaged in a glass vial sealed with a rubber stopper, or in a pre-filled-syringe, or in a cartridge or in a glass ampule, preferably the composition is packaged in a glass vial sealed with a rubber stopper.

In another embodiment, said pharmaceutical composition is to be parenterally administered to a human.

In another embodiment, said pharmaceutical composition is to be used as a medicament.

In another embodiment, said pharmaceutical composition is to be used in the treatment of dementia, cognitive impairment, Alzheimer or methemoglobinemia, preferably for the treatment of methemoglobinemia.

In another embodiment, the compositions show assay values within 95%-105% and total organic impurity content below 3% after 6 months storage under 40°C/75% RH packaged in glass vials with rubber stopper.

It is further disclosed, a sterile aqueous pharmaceutical composition comprising methylene blue as active ingredient, water, Azure B impurity and at least one metal, wherein the Azure B impurity content of said composition ranges from 2.0% to 3.0% with respect to the active ingredient, wherein the total metal content of said pharmaceutical composition is above 20 ppm, wherein the pH of said composition is below 4.0 and wherein the composition is free from pH adjusters is disclosed.

It is further disclosed, a sterile aqueous pharmaceutical composition comprising methylene blue as active ingredient at a concentration of 5 mg/mL, water, Azure B impurity and at least one metal, wherein the Azure B impurity content of said composition ranges from 2.0% to 3.0% with respect to the active ingredient, wherein the total metal content of said pharmaceutical composition is above 20 ppm, wherein the pH of said composition is below 4.0 and wherein the composition is free from pH adjusters is disclosed.

It is further disclosed, a sterile aqueous pharmaceutical composition consisting of methylene blue as active ingredient at a concentration of 5 mg/mL, water, methylene blue impurities and at least one metal, wherein the Azure B impurity content of said composition ranges from 2.0% to 3.0%, wherein the total metal content of said pharmaceutical composition is above 20 ppm, and wherein the pH of said composition is below 4.0.

In another embodiment, the composition further comprises not more than 0.20% of Azure A and not more than 0.20% of Azure C.

All the embodiments of the first aspect of the invention may be combined between them.

In a second aspect of the invention, a process for preparing said pharmaceutical composition is disclosed.

In an embodiment of the second aspect of the invention, a process for preparing the pharmaceutical composition of the first aspect of the invention comprising the steps of:
a. weighing the methylene blue,
b. in a reactor, dissolving and mixing methylene blue of step a. in water to obtain a solution
c. optionally, filtering the obtained solution,
d. filling the solution into a suitable pharmaceutical container,
e. sterilising the obtained solution.

In a further embodiment, the methylene blue in step a. has a purity below 98% and an Azure B content of at least 2%.

In a further embodiment, the reactor of step b. is a reactor made of metal, preferably made of stainless steel.

In a further embodiment, the filtration of step c. is filtered through a 0.2 µm pore size filter.

In a further embodiment, the filtration of step c. is performed twice.

In a further embodiment, the bioburden of the solution before any filtration is higher than 10 CFU/ 100 mL. In case the bioburden before filtration is not more than 10 CFU/ 100 mL. the filtration stages may be waived.

In a further embodiment, step e. is performed by terminal sterilisation and preferably reaching at least 110°C for not less than 10 minutes, more preferably reaching at least 120°C for not less than 10 minutes.

In a further embodiment, the terminal sterilisation of step e. is performed in an autoclave.

In a further embodiment, the aqueous solution reaches a temperature inside the reactor of at least 20°C.

In a further embodiment, the suitable pharmaceutical container of step e. is a glass vial sealed with a rubber stopper.

In a further embodiment, the vial is blanketed with nitrogen before sealing.

In a further embodiment, the sterilisation process reaches at least 121°C for at least 10 minutes, preferably 20 minutes.

In a further embodiment the sterilisation process is maintained between 121°C and 124°C in an autoclave during 20 minutes.

The embodiments of the second aspect of the invention may be combined between them to obtain the composition according to the first aspect of the invention. A preferred combination of embodiments is as follows:
A process for preparing a sterile aqueous pharmaceutical composition comprising methylene blue as active ingredient wherein the Azure B impurity content of said composition ranges from 2.0% to 3.0% and the pH of said composition is from 3.2 to 3.9 comprising the steps of:
a. weighing the methylene blue having a purity below 98% and an Azure B content of at least 2%,
b. dissolving and mixing in water methylene blue in a stainless steel reactor to obtain a solution,
c. optionally, filtering the obtained solution,
d. filling the solution into a suitable pharmaceutical container,
e. sterilising the obtained solution, preferably by terminal sterilisation and preferably reaching at least 110°C for not less than 10 minutes, more preferably reaching at least 120°C for not less than 10 minutes.

In another possible combination of the embodiments of the second aspect of the invention, the process is as follows:
1. Procedure of weighing and sampling methylene blue
   1. Transfer the active ingredient methylene blue to a controlled area and weigh the active ingredient to reach a final concentration of 5 mg/mL (weight 5 mg of methylene blue or any of its hydrates for each mL of solution to be manufactured). Protect the active ingredient from light during the whole manufacturing process.
2. Preparation of the solution
   1. Add water for injection (approximately 88% of the batch size volume)
   2. Add slowly the total amount of methylene blue sodium into the vessel under continuous stirring until the total dissolution of the API.
   3. Once the active ingredient is completely dissolved, set (if necessary) the temperature of the solution at 22 ± 3°C.
   4. Make up to the final volume with water for injection
   5. Stir the solution for not less than 20 minutes in order to ensure complete homogenization of the solution.
3. Filtration Stage I
   1. Transfer the solution from the stainless steel compounding vessel to a stainless steel holding vessel through a 0.2 µm pore size filter.
4. Filtration Stage II
   1. Transfer the solution from the stainless steel holding vessel to a stainless steel filling vessel through a 0.2 µm pore size filter.
5. Filling of the vials
   1. Fill aseptically 1 mL, 2 mL or 10 mL of the active solution into 10 mL type I clear glass vials and close them with a stopper and an aluminium seal.
6. Terminal sterilisation
   1. Place the filled vials in the autoclave
   2. Sterilise for 20 minutes between 121°C and 124°C.
   3. Cool down the sterilised vials to room temperature.
   4. Perform visual inspection of the vials and store them protected from light.

In a third aspect of the invention, an industrial pharmaceutical batch of the composition of the first aspect manufactured by the process of the second aspect is disclosed. Said pharmaceutical batch ranges between 15L and 120L of methylene blue aqueous solution.

In a further embodiment, said industrial pharmaceutical batch is packaged in glass vials, ampoules or pre filled syringes.

In another embodiment, each presentation contains 2 mL or 10 mL.

In a preferred embodiment, the pharmaceutical batch yields 13636 vials of the 2 mL presentation.

In a preferred embodiment, the pharmaceutical batch yields 9389 vials of the 10 mL presentation.

### EXAMPLES

### I. Preparation of Methylene Blue Injection USP compositions at 5 mg/mL

Methylene blue pharmaceutical compositions 1 to 7 for parenteral administration were prepared. General preparation method is described below:
- Weight the necessary amount of methylene blue to reach a final concentration of 5 mg/ml
- Add water for injection in a stainless steel vessel
- Add slowly the total amount of methylene blue into the vessel under continuous stirring until the total dissolution of the methylene blue
- Add water for injection until to reach the final volume
- Stir the resulting solution to ensure complete homogenization of the solution
- Filter twice the solution through a 0.2 µm pore size filter
- Fill vials with the obtained solution and seal them with a rubber stopper
- Place the filled vials in the autoclave and sterilise them

### II. Analysis of the prepared compositions

Compositions 1 to 7 were prepared as described in previous point I. The compositions 1, 2, 3, 6, 7 are not in the scope of the claims.

| | Comp 1 | Comp 2 | Comp 3 | Comp 4 | Comp 5 | Comp 6 | Comp 7 |
|---|---|---|---|---|---|---|---|
| Reactor material | Stainless steel | Glass | Stainless steel | Stainless steel | Stainless steel | Stainless steel | Stainless steel |
| pH | 4.1 | 4.6 | 4.6 | 3.2 | 3.9 | 3.8 | 3.8 |
| Azure B impurity (%) | 1.9 | 2.4 | 2.5 | 2.3 | 2.4 | 2.6 | 2.5 |
| Total impurity content (%) | 1.9 | 2.4 | 2.5 | 2.3 | 2.4 | 2.5 | 2.5 |
| Metal content (ppm) | 23.63 | 131.0 | 105.2 | 192.8 | 131.2 | 35.4 | 140.1 |
| Iron content (ppm) | ND | 56 | 52 | 94 | 102 | ND | 112 |
| Aluminium content (ppm) | ND | 8 | 8 | 26 | 24 | 16 | ND |
| Assay (%) | 94.7 | 96.6 | 95.6 | 100.1 | 100.1 | 99.7 | 96.2 |

| | | | | | | | |
|---|---|---|---|---|---|---|---|
| ND: Not detected | | | | | | | |

Compositions 2, 3, 6 and 7 were adjusted for pH to obtain comparative examples. Anhydrous citric acid and sodium citrate dihydrate were used at q.s. to arrive at the pH shown in the table.

Compositions 4 and 5 were not adjusted for pH and consist of methylene blue and water. The metal and impurity content of the active ingredient did not increase during manufacturing.

### III. Stability of the prepared compositions

During manufacturing of all the compositions, we did not identify a significant increase in the total organic impurities before and after manufacturing. That is, the organic impurities carried by the active ingredient did not increase during manufacturing nor during a stability period of 6 months stored at 40°C/75% RH or a period of 24 months stored at 25°C/60% RH.

### IV. General formula of the prepared compositions

| Ingredient | Quantity |
|---|---|
| Methylene blue | 5 mg/mL |
| Water for Injection | q.s. to 1 mL |

Commercial batch size manufactured: 15 Liters
Presentations packaged: 2 mL glass vial and 10 mL glass vial

## Claims

1. A sterile aqueous pharmaceutical composition consisting of methylene blue as active ingredient and water, wherein
a. the Azure B impurity content of said composition ranges from 2.0% to 3.0% and,
b. the pH of said composition is from 3.2 to 3.9, preferably from 3.3 to 3.8 and,
c. the Iron (Fe) content of said pharmaceutical composition is above 20 ppm, preferably above 50 ppm, more preferably above 100 ppm, even more preferably above 120 ppm.

2. The pharmaceutical composition according to the preceding claim wherein the Azure B impurity content of said composition ranges from 2.1% to 3.0%, preferably from 2.3% to 3.0%.

3. The pharmaceutical composition according to any of the preceding claims wherein the metal content in the composition is above 120 ppm, preferably above 130 ppm, more preferably above 150 ppm.

4. The pharmaceutical composition according to any of the preceding claims wherein the pharmaceutical composition is packaged in a glass vial, or in a pre-filled-syringe, or in a cartridge or in an ampule, preferably the composition is packaged in a glass vial and sealed with a rubber stopper.

5. The pharmaceutical composition according to any of the previous claims wherein the composition is to be parenterally administered to a human.

6. The pharmaceutical composition according to any of the preceding claims for use as a medicament.

7. The pharmaceutical composition according to any of the preceding claims for the use in the treatment of dementia, cognitive impairment, Alzheimer or methemoglobinemia.

8. A process for preparing a pharmaceutical composition according to any of the preceding claims comprising the steps of:
a. weighing the methylene blue,
b. in a reactor, dissolving and mixing methylene blue of step a. in water to obtain a solution,
c. optionally, filtering the obtained solution,
d. filling the solution into a suitable pharmaceutical container,
e. sterilising the obtained solution.

9. A process according to the preceding claim wherein the reactor material is metallic, preferably stainless steel.

10. A process according to any of the two preceding claims wherein the solution of step c) is filtered through a 0.2 µm pore size filter.

11. The process according to the preceding claim, wherein the filtration is performed twice.

12. A process according to any of the **four** preceding claims, wherein the suitable pharmaceutical container is a glass vial sealed with a rubber stopper.

13. A process according to any of the **five** preceding claims, wherein the pH of the aqueous solution ranges between 3.2 and 3.9.

## Patentansprüche

1. Sterile wässrige pharmazeutische Zusammensetzung, bestehend aus Methylenblau als Wirkstoff und Wasser, wobei
a. der Gehalt der Zusammensetzung an Azur B-Verunreinigung im Bereich von 2,0 % bis 3,0 % liegt und
b. der pH-Wert der Zusammensetzung 3,2 bis 3,9, vorzugsweise 3,3 bis 3,8 beträgt und
c. der Eisen-(Fe)-Gehalt der pharmazeutischen Zusammensetzung über 20 ppm, vorzugsweise über 50 ppm, bevorzugter über 100 ppm, noch bevorzugter über 120 ppm liegt.

2. Pharmazeutische Zusammensetzung nach dem vorhergehenden Anspruch, wobei der Gehalt der Zusammensetzung an Azur B-Verunreinigung im Bereich von 2,1 % bis 3,0 %, vorzugsweise von 2,3 % bis 3,0 % liegt.

3. Pharmazeutische Zusammensetzung nach einem der vorhergehenden Ansprüche, wobei der Metallgehalt in der Zusammensetzung über 120 ppm, vorzugsweise über 130 ppm, bevorzugter über 150 ppm liegt.

4. Pharmazeutische Zusammensetzung nach einem der vorhergehenden Ansprüche, wobei die pharmazeutische Zusammensetzung in einem Glasfläschchen, in einer Fertigspritze, in einer Kartusche oder in einer Ampulle verpackt ist, vorzugsweise die Zusammensetzung in einem Glasfläschchen verpackt und mit einem Gummistopfen verschlossen ist.

5. Pharmazeutische Zusammensetzung nach einem der vorhergehenden Ansprüche, wobei die Zusammensetzung einem Menschen parenteral zu verabreichen ist.

6. Pharmazeutische Zusammensetzung nach einem der vorhergehenden Ansprüche zur Verwendung als Arzneimittel.

7. Pharmazeutische Zusammensetzung nach einem der vorhergehenden Ansprüche zur Verwendung bei der Behandlung von Demenz, kognitiver Beeinträchtigung, Alzheimer oder Methämoglobinämie.

8. Verfahren zur Herstellung einer pharmazeutischen Zusammensetzung nach einem der vorhergehenden Ansprüche, umfassend die Schritte:
a. Einwiegen des Methylenblaus,
b. Lösen und Mischen des Methylenblaus aus Schritt a. in Wasser in einem Reaktor, um eine Lösung zu erhalten,
c. gegebenenfalls Filtrieren der erhaltenen Lösung,
d. Abfüllen der Lösung in einen geeigneten pharmazeutischen Behälter,
e. Sterilisieren der erhaltenen Lösung.

9. Verfahren nach dem vorhergehenden Anspruch, wobei das Reaktormaterial metallisch ist, vorzugsweise Edelstahl.

10. Verfahren nach einem der beiden vorhergehenden Ansprüche, wobei die Lösung aus Schritt c) durch einen Filter mit einer Porengröße von 0,2 µm filtriert wird.

11. Verfahren nach dem vorhergehenden Anspruch, wobei die Filtration zweimal durchgeführt wird.

12. Verfahren nach einem der vier vorhergehenden Ansprüche, wobei der geeignete pharmazeutische Behälter ein mit einem Gummistopfen verschlossenes Glasfläschchen ist.

13. Verfahren nach einem der fünf vorhergehenden Ansprüche, wobei der pH-Wert der wässrigen Lösung zwischen 3,2 und 3,9 liegt.

## Revendications

1. Composition pharmaceutique aqueuse stérile consituée de bleu de méthylène en tant que principe actif et de l'eau, dans laquelle :
a. la teneur en impureté Azur B de ladite composition va de 2,0 % à 3,0 %, et
b. le pH de ladite composition est de 3,2 à 3,9, de préférence de 3,3 à 3,8, et
c. la teneur en fer (Fe) de ladite composition pharmaceutique est supérieure à 20 ppm, de préférence supérieure à 50 ppm, plus préférablement supérieure à 100 ppm, encore plus préférablement supérieure à 120 ppm.

2. Composition pharmaceutique selon la revendication précédente, dans laquelle la teneur en impureté Azur B de ladite composition va de 2,1 % à 3,0 %, de préférence de 2,3 % à 3,0 %.

3. Composition pharmaceutique selon l'une quelconque des revendications précédentes, dans laquelle la teneur en métal dans la composition est supérieure à 120 ppm, de préférence supérieure à 130 ppm, plus préférablement supérieure à 150 ppm.

4. Composition pharmaceutique selon l'une quelconque des revendications précédentes, dans laquelle la composition pharmaceutique est conditionnée dans un flacon en verre, ou dans une seringue préremplie, ou dans une cartouche ou dans une ampoule, de préférence la composition est conditionnée dans un flacon en verre et scellée par un bouchon en caoutchouc.

5. Composition pharmaceutique selon l'une quelconque des revendications précédentes, dans laquelle la composition est destinée à être administrée par voie parentérale à un être humain.

6. Composition pharmaceutique selon l'une quelconque des revendications précédentes pour son utilisation comme médicament.

7. Composition pharmaceutique selon l'une quelconque des revendications précédentes pour son utilisation dans le traitement de la démence, d'un trouble cognitif, de la maladie d'Alzheimer ou de la méthémoglobinémie.

8. Procédé de préparation d'une composition pharmaceutique selon l'une quelconque des revendications précédentes, comprenant les étapes de :
a. pesage du bleu de méthylène,
b. dans un réacteur, dissolution et mélange du bleu de méthylène de l'étape a. dans de l'eau pour obtenir une solution,
c. éventuellement, filtration de la solution obtenue,
d. remplissage d'un récipient pharmaceutique approprié avec la solution,
e. stérilisation de la solution obtenue.

9. Procédé selon la revendication précédente, dans lequel le matériau du réacteur est métallique, de préférence l'acier inoxydable.

10. Procédé selon l'une quelconque des deux revendications précédentes, dans lequel la solution de l'étape c) est filtrée à travers un filtre ayant une taille de pores de 0,2 µm.

11. Procédé selon la revendication précédente, dans lequel la filtration est réalisée deux fois.

12. Procédé selon l'une quelconque des quatre revendications précédentes, dans lequel le récipient pharmaceutique approprié est un flacon en verre scellé par un bouchon en caoutchouc.

13. Procédé selon l'une quelconque des cinq revendications précédentes, dans lequel le pH de la solution aqueuse est compris entre 3,2 et 3,9.
